# EUROPEAN PATENT APPLICATION

(11) **EP 3 620 538 A1**
(43) Date of publication of application: **11.03.2020**
(21) Application number: 18192363.2
(22) Date of filing: 04.09.2018
(51) Int. Cl.: C12Q 1/6886

(54) **MEANS AND METHODS FOR STRATIFYING PROSTATE CANCER PATIENTS**

(71) Applicant: Jessenius Faculty of Medicine of Comenius University in Martin, 036 01 Martin (SK)
(72) Inventor: BALUCHOVÁ, Katarína, 811 05 Bratislava (SK); KLIMENT, Ján, 036 01 Martin (SK)
(74) Representative: Schiweck Weinzierl Koch Patentanwälte Partnerschaft mbB

(57) **Abstract**

The present invention is in the field of molecular biology. The present invention provides a method for stratifying a subject suffering from prostate cancer. The present invention further provides primers and a kit allowing stratification of a subject suffering from prostate cancer.

## Description

### I. Background

Prostate cancer (PC) is the leading and the second most frequently diagnosed oncological disorder in European and in Slovakian men, respectively and the second and the third-leading cause of associated deaths in these subsets (https://ecis.jrc.ec.europa.eu, accessed on 27/06/2018). As such PC management has high medical priority, yet diagnostic and therapeutic options are dependent on and limited to the stage of the disease (Mottet, N., et al. EAU-ESTRO-SIOG Guidelines on Prostate Cancer. Part 1: Screening, Diagnosis, and Local Treatment with Curative Intent. (2017), Eur. Urol., 71: 618-629; Cornford, P., et al. EAU-ESTRO-SIOG Guidelines on Prostate Cancer. Part II: Treatment of Relapsing, Metastatic, and Castration-Resistant Prostate Cancer. (2017), Eur. Urol., 71: 630-642). Approximately 60 to 70 % of men are diagnosed with localised PC are hence offered sequentially active surveillance, radical prostatectomy, radiotherapy combined with or without hormonal treatment and brachytherapy. The majority of patients with localised disease respond well to the treatment and almost all live longer than 5 years. The remaining PC patients of 30 to 40% have at diagnosis advanced stages of PC with the minority of 8 to 11 % having metastatic disease (Crawford ED et al., (2017), Urol. Oncol., 35S: S1-S13). Metastatic disease is at the onset hormone-sensitive and responds well to common hormonal therapies referred to as androgen deprivation therapies (ADT). The aim of ADP is to supress testosterone to castration levels and this can be achieved with LHRH agonists or antagonists or through surgery. At a later stage of the disease, however, resistance to ADT invariably develops and PC becomes castration-resistant (CR) despite continuous ADT. Especially, patients with early-onset PC and those progressing rapidly are at risk developing aggressive life-threatening CRPC.

mCRPC is the late and frequently lethal stage of PC being diagnosed in 8 to 11 % of incoming patients and progressing from earlier stages of the disease to mCRPC in 10 to 20 % of PC patients (Crawford ED et al., (2017), Urol. Oncol., 35S: S1-S13; Kirby M et al., (2011), Int J Clin Pract., 65(11): 1180-92). Therapeutic options of mCRPC with median survival time of approximately 15 to 36 months include chemotherapy, radiotherapy, surgical interventions, immunotherapy and ADT that interferes with androgen signalling pathways (Cornford, P., et al. EAU-ESTRO-SIOG Guidelines on Prostate Cancer. Part II: Treatment of Relapsing, Metastatic, and Castration-Resistant Prostate Cancer. (2017), Eur. Urol., 71: 630-642). More recently, ADT in mCRPC patients was extended to *abiraterone* and *enzalutamide,* collectively referred to as ARSi, potent androgen receptor signalling inhibitors of androgen biosynthesis and AR binding, respectively (Moreira RB et al., (2017). Oncotarget, 8(48): 84572-84578). Unfortunately, these drugs are effective not in all mCRPC patients, since primary resistance operates in 20 to 40 % of mCRPC patients and secondary resistance develops invariably during the course of treatment (Antonarakis ES et al., (2014). N Engl J Med., 371(11): 1028-38; Scher HI et al., (2012). N Engl J Med., 367(13): 1187-97; de Bono JS et al., (2011). N Engl J Med., 364(21): 1995-2005).

Whereas secondary resistance cannot be effectively avoided at the present, primary resistance is linked to the expression of splicing variant of AR, referred to as androgen receptor splice variant 7 (AR-V7) and thus can be predicted (Boudadi K, Antonarakis ES, (2016). Clin Med Insights Oncol. 10(1): 1-9; Antonarakis ES et al., (2017). J Clin Oncol., 35(19): 2149-2156). Accordingly, AR-V7 expression serves as a biomarker for elucidating the sensitivity and resistance to *abirateronelenzalutamide-* and *taxane-based* therapies in mCRPC patients. As a result AR-V7 positive mCRPC patients, in line with the recommendations of the international urological societies, disqualify for the treatment of ARSi (Cornford, P., et al. EAU-ESTRO-SIOG Guidelines on Prostate Cancer. Part II: Treatment of Relapsing, Metastatic, and Castration-Resistant Prostate Cancer. (2017), Eur. Urol., 71: 630-642). Instead and more importantly before the application and subsequent failure of the ARSi-therapy, they can be offered life-extending and quality of life improving therapies at an earlier time-point in the form of chemotherapy. Chemotherapy exemplified by *docetaxel* and *cabazitaxel* reduces debilitating symptoms at the onset of mCRPC improving time-management of the disease and reducing costs to health care service providers.

The common testing materials for expression of AR-V7 in mCRPC patients include open surgery biopsies and transrectal ultrasound-guided biopsies also referred to as solid biopsies and obtained from invasive surgical procedures. More recently less invasive procedures such as phlebotomy are used to access tumour cells circulating in the peripheral blood (CTC). CTC are are an attractive option for deciphering the AR-V7 status in mCRPC patients, since they improve patient compliance, save time, personal and costs needed for obtaining accessible representative tumour-derived material (Zhang T, Armstrong AJ, (2016). Curr Oncol Rep., 18(3): 1-8). CTC are also the liquid biopsy biomarkers that unveil evolution of the disease, since they unlike solid biopsies, reflect the real-time progression and heterogeneity of the malignant process.

Current AR-V7 tests include among others quantitative real-time PCR (Antonarakis ES et al., (2014). N Engl J Med., 371(11): 1028-38; Lokhandwala PM et al., (2017). J Mol Diagn. 19(1): 115-125), droplet digital PCR (Ma Y et al., (2016). Int J Mol Sci., 17(8): 1-11), nanofluidic quantitative RT-PCR (Škereňová M et al., Gene Expression Analysis of Immunomagnetically Enriched Circulating Tumor Cell Fraction in Castration-Resistant Prostate Cancer (2018). Mol Diagn Ther., ahead of print), NGS-based screenings (Robinson D et al., (2015). Cell, 161(5): 1215-1228), Western blot (Welti J et al., (2016). Eur Urol., 70(4): 599-608) and immunochemistry (Qu Y et al., (2015). Sci Rep., 5 (7654): 1-6) among others. Yet all these methods are complex, time-consuming, laborious and expensive and thus unsuitable for clinical laboratories. Accordingly, there is an unmet need for a simple, reliable, cost-effective and clinically applicable method that allows stratifying mCRPC patients according to their AR-V7 expression status to ARSi responders and ARSi non-responders.

The objective of the present invention is therefore to introduce a cost-effective, reliable, simple and clinically applicable method for stratifying castration-resistant prostate cancer patients to androgen receptor signalling inhibitors responders and non-responders. This objective is achieved by the embodiments and subject matter contained in the claims and in the following description, which are illustrated by the examples and figures.

### II. Definitions

The following list defines terms, phrases, and abbreviations used throughout the instant specification. All terms listed and defined herein are intended to encompass all grammatical forms.

It must be noted that as used herein, the singular forms "a", "an", and "the", include plural references unless the context clearly indicates otherwise. Thus, for example, reference to "an expression cassette" includes one or more of the expression cassettes disclosed herein and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the present invention.

The term "and/or" wherever used herein includes the meaning of "and", "or" and "all or any other combination of the elements connected by said term". For example, A, B and/or C means A, B, C, A+B, A+C, B+C and A+B+C.

The term "about" or "approximately" as used herein means within 20 %, preferably within 10%, and more preferably within 5% of a given value or range. It includes also the concrete number, e.g., about 20 includes 20.

The term "less than", "more than" or "larger than" includes the concrete number. For example, less than 20 means ≤20 and more than 20 means ≥20.

Further, in describing representative embodiments of the present invention, the specification may have presented the method and/or process of the present invention as a particular sequence of steps. However, to the extent that the method or process does not rely on the particular order of steps set forth herein, the method or process should not be limited to the particular sequence of steps described. As one of ordinary skill in the art would appreciate, other sequences of steps may be possible. Therefore, the particular order of the steps set forth in the specification should not be construed as limitations on the claims. In addition, the claims directed to the method and/or process of the present invention should not be limited to the performance of their steps in the order written, and one skilled in the art can readily appreciate that the sequences may be varied and still remain within the spirit and scope of the present invention.

It should be understood that this invention is not limited to the particular methodology, protocols, material, reagents, and substances, etc., described herein. The terminologies used herein are for the purpose of describing particular embodiments only and are not intended to limit the scope of the present invention, which is defined solely by the claims/items.

All publications and patents cited in this disclosure are incorporated by reference in their entirety. To the extent the material incorporated by reference contradicts or is inconsistent with this specification, the specification will supersede any such material.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or sometimes when used herein with the term "having". When used herein "consisting of" excludes any element, step, or ingredient not specified in the claim element. When used herein, "consisting essentially of" does not exclude materials or steps that do not materially affect the basic and novel characteristics of the claim. In each instance herein any of the terms "comprising", "consisting essentially of" and "consisting of" may be replaced with either of the other two terms.

Unless otherwise defined herein, scientific and technical terms used in connection with the present invention shall have the meanings that are commonly understood by those of ordinary skill in the art. Further, unless otherwise required by context, singular terms shall include pluralities and plural terms shall include the singular. The methods and techniques of the present invention are generally performed according to conventional methods well-known in the art. Generally, nomenclatures used in connection with techniques of biochemistry, enzymology, molecular and cellular biology, microbiology, genetics and protein and nucleic acid chemistry and hybridization described herein are those well-known and commonly used in the art.

The methods and techniques of the present invention are generally performed according to conventional methods well-known in the art and as described in various general and more specific references that are cited and discussed throughout the present specification unless otherwise indicated. See, e. g., Sambrook et al., Molecular Cloning: A Laboratory Manual, 3rd ed., Cold Spring Harbor Laboratory Press, Cold Spring Harbor, N. Y. (2001); Ausubel et al., Current Protocols in Molecular Biology, J, Greene Publishing Associates (1992, and Supplements to 2002); Handbook of Biochemistry: Section A Proteins, Vol I (1976) CRC Press; Handbook of Biochemistry: Section A Proteins, Vol II 1976 CRC Press. The nomenclatures used in connection with, and the laboratory procedures and techniques of, molecular and cellular biology, protein biochemistry, enzymology and medical and pharmaceutical chemistry described herein are those well-known and commonly used in the art.

"Identity" is a property of sequences that measures their similarity or relationship. The term "sequence identity" or "identity" as used in the present disclosure means the percentage of pair-wise identical residues-following (homologous) alignment of a sequence of a nucleic acid of the disclosure with a sequence in question-with respect to the number of residues in the longer of these two sequences. Sequence identity is measured by dividing the number of identical nucleic acid residues by the total number of residues and multiplying the product by 100.

The term "isolated" means a substance in a form or environment that does not occur in nature. Non-limiting examples of isolated substances include (1) any non-naturally occurring substance; (2) any substance including, but not limited to, any enzyme, variant, nucleic acid, protein, peptide or cofactor, that is at least partially removed from one or more or all of the naturally occurring constituents with which it is associated in nature; (3) any substance modified by the hand of man relative to that substance found in nature, e.g., cDNA made from mRNA; or (4) any substance modified by increasing the amount of the substance relative to other components with which it is naturally associated (e.g., recombinant production in a host cell; multiple copies of a gene encoding the substance; and use of a stronger promoter than the promoter naturally associated with the gene encoding the substance).

Isolation of a desired population of cells may in some embodiments include general cell enrichment techniques such as centrifugation, filtration or cell chromatography. Generally, isolating or enriching a desired population of cells may be carried out according to any desired technique known in the art. In some embodiments isolation of a desired population of cells may include the use of a commercially available cell isolation kit. Methods to isolate cells are known to the person skilled in the art. Methods for isolating CTC are further described herein.

The term "nucleotide sequence" or "nucleic acid sequence" used herein refers to either DNA or RNA. "Nucleic acid sequence" or "polynucleotide sequence" or simply "polynucleotide" refers to a single or double-stranded polymer of deoxyribonucleotide or ribonucleotide bases read from the 5' to the 3' end. It includes both self-replicating plasmids, infectious polymers of DNA or RNA, and non-functional DNA or RNA. The use of the terms " 5' " and " 3' " is a convention used to describe features of a nucleotide sequence related to either the position of genetic elements and/or the direction of events (5' to 3'), such as e.g., transcription by RNA polymerase or translation by the ribosome which proceeds in 5' to 3' direction. Synonyms are upstream (5') and downstream (3'). Conventionally, nucleotide sequences, gene maps, vector cards and RNA sequences are drawn with 5' to 3' from left to right or the 5' to 3' direction is indicated with arrows, wherein the arrowhead points in the 3' direction. Accordingly, 5' (upstream) indicates genetic elements positioned towards the left hand side, and 3' (downstream) indicates genetic elements positioned towards the right hand side, when following this convention.

By "gene" is meant a unit of inheritance that occupies a specific locus on a chromosome and that is a segment of nucleic acid associated with a biological function. A gene encompasses transcriptional and/or translational regulatory sequences as well as a coding region. Besides a coding sequence a gene may include a promoter region, a cis-regulatory sequence, a non-expressed DNA segment that is a specific recognition sequence for regulatory proteins, a non-expressed DNA segment that contributes to gene expression, a DNA segment designed to have desired parameters, or combinations thereof. A gene can be obtained by a variety of methods, including cloning from a biological sample, synthesis based on known or predicted sequence information, and recombinant derivation of an existing sequence.

The term "expressing" as used herein refers to the synthesis of a gene product encoded by a polynucleotide. In context of a polypeptide "expressing" means when a polynucleotide is transcribed to mRNA and the mRNA is translated to a polypeptide. The term "expressing" in context of a RNA means when a DNA is transcribed to RNA, e.g., a tRNA.

The term "amino acid" or "natural amino acid" are used interchangeably herein and refer to naturally occurring and synthetic amino acids, as well as amino acid analogs and amino acid mimetics that function in a manner similar to the naturally occurring amino acids. Naturally occurring amino acids are those encoded by the genetic code, as well as those amino acids that are later modified, e.g., hydroxyproline, γ-carboxyglutamate, and O-phosphoserine. Amino acid analogs refers to compounds that have the same basic chemical structure as a naturally occurring amino acid, i.e., a carbon that is bound to a hydrogen, a carboxyl group, an amino group, and an R group, e.g., homoserine, norleucine, methionine sulfoxide, methionine methyl sulfonium. Such analogs have modified R groups (e.g., norleucine) or modified peptide backbones, but retain the same basic chemical structure as a naturally occurring amino acid. However, such an analog is not to be confused with an unnatural amino acid, which comprises one or more amino acid residues fused to the R group of an amino acid. Amino acid mimetics refers to chemical compounds that have a structure that is different from the general chemical structure of an amino acid, but that function in a manner similar to a naturally occurring amino acid.

The terms "polypeptide" and "protein" are interchangeably used. The term "polypeptide" refers to a protein or peptide that contains two or more amino acids, typically at least 3, preferably at least 20, more preferred at least 30, such as at least 50 amino acids. Accordingly, a polypeptide comprises an amino acid sequence, and, thus, sometimes a polypeptide comprising an amino acid sequence is referred to herein as a "polypeptide comprising a polypeptide sequence". Thus, herein the term "polypeptide sequence" is interchangeably used with the term "amino acid sequence".

### III. Description of the Figures

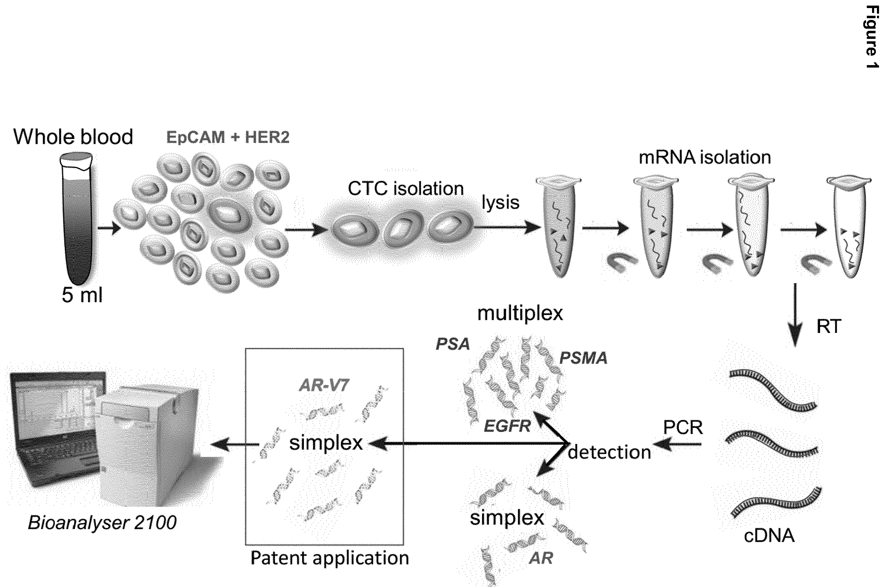

**Figure 1****.** Schematic diagram used to test AR-V7 expression in CTC from mCRPC patients alongside the expression of other tumour-associated genes.

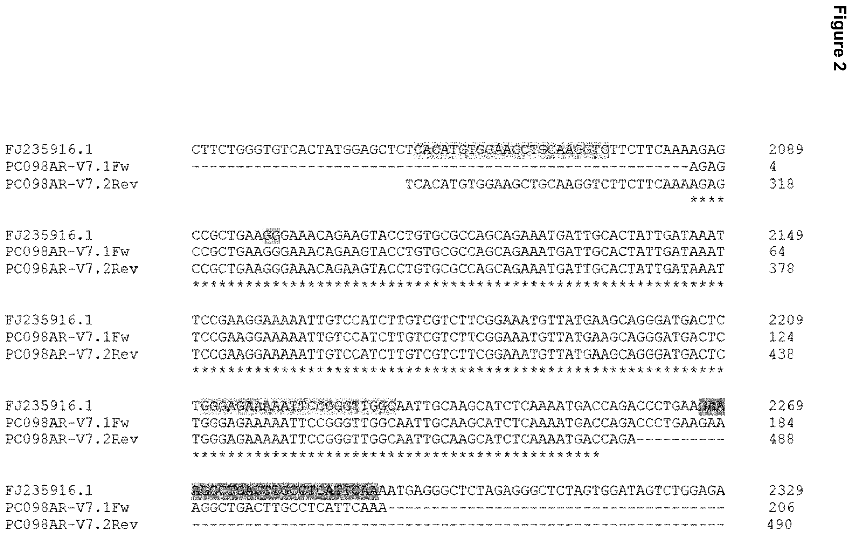

**Figure 2****.** Sequence alignment of Genbank reference AR-V7 sequence FJ235916.1 against sequenced patient's material PC098 using primers AR-V7.1 Fw, AR-V7.1 Rev and AR-V7.2Rev. Sequence of primers are highlighted in yellow (AR-V7.1Fw and AR-V7.1 Rev) and in green (AR-V7.2Rev). The bases GG at AR-V7 exon 2 and exon 3 junction and the bases GA at exon 3 and exon CE3 junction are shown in grey.

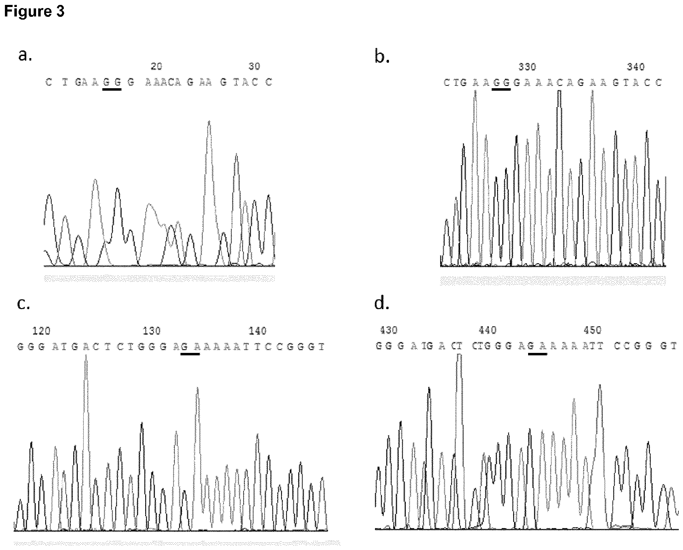

**Figure 3****.** Four-colour Sanger sequencing chromatograms of RT-PCR products produced for one patient targeting AR-V7 region using primers to cover the junctions between AR-V7 exon 2 and exon 3 (a.) using the primer AR-V7.1 Fw and (b.) the primer AR-V7.1 Rev and to cover the junction between AR-V7 exon 3 and exon CE3 (c.) using the primer AR-V7.1 Fw and (d.) the primer AR-V7.2 Rev. Exon junctions are underlined.

### IV. Detailed description of the invention

In patients with metastatic castration-resistant prostate cancer (mCRPC) resistance to treatment with potent androgen receptor signaling inhibitors, such as *abiraterone* and *enzalutamide,* is linked to the expression of androgen receptor splice variant 7 (AR-V7), and can thus be predicted. Consequently, the AR-V7 expression renders mCRPC patients resistant to *abiraterone* and *enzalutamide* and these as a result may be treated with e.g., taxane-based therapies. Therefore, it would be desirable to have a clinically applicable and inexpensive method to determine AR-V7 status actionable for matched therapies in mCRPC patients within a short period of time. However, AR-V7 expression is currently detected using complex, time-consuming, laborious and expensive methods hindering a broad application in clinical laboratories. Accordingly, there is an unmet need for a simple, reliable and cost-efficient stratifying method deciphering the AR-V7 status of a mCRPC patient. The present inventors meet this need by providing a stratification method that employs qualitative PCR to determine the AR-V7 status of a mCRPC patient. The present inventors surprisingly discovered that such a test is as sensitive and as reliable as the more sophisticated, laborious, time-consuming and expensive AR-V7 testing platforms. The method according to the invention is cost-efficient, reliable, simple and can thus be directly applied for stratifying mCRPC patients in clinical settings.

In particular, the present invention provides a method for stratifying a subject suffering from prostate cancer, comprising:
(i) isolating a prostate cancer cell from a biopsy obtained from the subject;
(ii) obtaining mRNA from the prostate cancer cell;
(iii) preparing cDNA from the mRNA obtained in step (ii); and
(iv) performing a qualitative PCR to determine the AR-V7 status of the cell.

The method is generally an *in vitro* or *ex vivo* method.

The term "AR-V7" as used herein relates to androgen-receptor splice variant 7. The androgen-receptor isoform encoded by splice variant 7 lacks the ligand-binding domain, which is the target of *abiraterone* and *enzalutamide,* but remains constitutively active as a transcription factor. The nucleic acid sequence of AR-V7 is set forth in SEQ ID NO: 6.

The term "determining the AR-V7 status" as used herein means the qualitative detection of the expression of AR-V7 in a prostate cancer cell. To this end, mRNA is isolated from the cell and reverse transcribed to cDNA, which serves as a template in the qualitative PCR reaction. Isolation of mRNA and subsequent generation of cDNA can be performed using any suitable method known in the art. For the reverse transcription, use is made of oligo-dT primers (preferably Oligo(dT)₂₅ primers) together with the enzyme reverse transcriptase (RT). The RT enzyme is preferably inactivated after the reverse transcription. Isolation of mRNA and reverse transcription to cDNA is further described in the Examples in detail. Thus, in case a prostate cancer cell contains mRNA encoding AR-V7, this will be transcribed to cDNA eventually resulting in amplification using qualitative PCR and AR-V7 specific primers. Such an AR-V7 specific amplification can be assessed using any suitable method known in the art, e.g., gel electrophoresis. In case specific amplification is detected, the AR-V7 status of the prostate cancer cell is marked as positive. However, the method according to the invention does not necessarily have to result in the positive detection of a nucleic acid, because in a prostate cancer cell, there could be no mRNA encoding AR-V7. Thus, in case no specific amplification is detected, the AR-V7 status of the prostate cancer cell is marked as negative. The "determining" with the aid of the method according to the invention therefore also comprises determining whether or not a mRNA encoding AR-V7 is contained in a prostate cancer cell. In any case, the method of the present invention does not require the calculation of a ratio, e.g., AR-full-length/AR-V7 expression ratio, as determined in the art.

A person skilled in the art knows how to isolate a prostate cancer cell from a biopsy. A prostate cancer cell can be isolated using any suitable method known in the art. Isolating circulating tumor cells from a prostate cancer patient is described herein in the Examples and in Antonarakis ES et al., (2014). N Engl J Med., 371(11): 1028-38, which is incorporated herein by reference in its entirety.

A "qualitative PCR" as used herein allows amplifying a segment of DNA and is usually performed using a DNA template (i.e., cDNA generated from mRNA isolated from a prostate cancer cell), a DNA polymerase, a DNA primer pair(s), dNTPs and a suitable buffer (comprising MgCl₂) allowing the polymerization reaction to take place. The result of a PCR can e.g., be analyzed using electrophoresis, allowing assessment whether amplification of the template occurred.

In a preferred embodiment of the invention, the biopsy is a solid biopsy. A solid biopsy that can be used according to the present invention can be obtained from invasive surgical procedures such as open surgery biopsies and transrectal ultrasound-guided biopsies.

In another preferred embodiment, the biopsy is a liquid biopsy. In a more preferred embodiment, the biopsy is peripheral blood obtained from a subject. Preferably, at least 1, 2, 3, 4, 5, 6, 7, 8, 9 or 10 ml of peripheral blood is used as liquid biopsy. More preferably, at least 5 ml of peripheral blood is used as liquid biopsy.

In case a liquid biopsy, such as peripheral blood, is used according to the method of the invention, the prostate cancer cell is preferably a circulating tumor cell (CTC). CTC are convenient source of tumor cells since they can be obtained easily without the need of surgical procedures. CTC also unveil evolution of the disease since they - unlike solid biopsies - reflect the real-time progress and heterogeneity of the malignant process. Preferably at least 2, 3, 4, 5, 6, 7, 8, 9, 10, 15, 20, 25, 30, 40, 50, 75 or 100 CTC are used in accordance with the method of the invention. More preferably, at least 5 CTC are used in accordance with the method of the invention.

A CTC can be isolated using any suitable method known in the art, e.g., using antibodies binding to CTC-specific cell surface markers. In a preferred embodiment magnetic beads coated with antibodies binding to EpCAM and/or HER-2 are used in accordance with the method of the present invention. Such beads are contained in the AdnaTest® system that is commonly used in the art and referred to as *ProstateCancerSelect* component of the AdnaTest® system. Thus, in a preferred embodiment CTC are isolated using the *ProstateCancerSelect* component of the AdnaTest® system. In a further preferred embodiment, the isolated CTC is identified by determining prostate cancer-associated gene expression of PSA, PSMA, EGFR and/or AR, wherein a circulating tumor cell expresses at least one of said genes. Preferably, prostate cancer-associated gene expression is determined using qualitative PCR (i.e., RT-PCR). Preferably, expression of actin or any other housekeeping gene is used as an internal control. In a preferred embodiment of the invention, tumor cells are identified using the *ProstateCancerDetect* component of the AdnaTest® system. Cancer-associated gene expression is commonly detected in the art using qualitative PCR (i.e., RT-PCR), e.g., using the *ProstateCancerDetect* component of the AdnaTest® system. Thus, before the present invention determining the AR-V7 status of a CTC it requires the consecutive steps of (i) isolating the CTC, (ii) identifying the CTC using RT-PCR and (iii) determining the AR-V7 status of the isolated CTC using e.g., quantitative real-time PCR, droplet digital PCR, nanofluidic quantitative RT-PCR, NGS-based screenings, Western blot or immunochemistry. In contrast to methods used in the art, the method of the present invention employs directly RT-PCR to determine the AR-V7 status of CTC and thus the same technique that is used to identify CTC. This allows for implementing the step of determining the AR-V7 status of the CTC in the step of identifying the CTC by simply adding a further PCR reaction. Thus, the method of the present invention advantageously allows identifying the CTC and determining its AR-V7 status simultaneously. Accordingly, the method of the present invention allows determining the AR-V7 status required for matched therapies in prostate cancer patients within a short period of time, unmatched by other methods used in the art.

In a further preferred embodiment, the prostate cancer is metastatic castration-resistant prostate cancer (mCRPC). mCRPC is the late and frequently lethal stage of PC, which has developed resistant to ADT.

In a preferred embodiment of the present invention, a qualitative PCR is not a quantitative PCR. In a further preferred embodiment, a qualitative PCR is not quantitative real-time PCR, droplet digital PCR or nanofluidic quantitative RT-PCR.

In a further preferred embodiment of the present invention, the qualitative PCR is performed using primers specifically amplifying a mRNA comprising the AR-V7 coding sequence. AR-V7 specific regions are regions spanning exons 2, 3 and the cryptic exon 3, i.e., junction between AR-V7 exon 2 and exon 3 and junction between AR-V7 exon 3 and exon CE3. Both junctions are shown in Figure 2 herein. Thus, primer pairs that can be used in accordance with the present invention preferably allow for amplification of a region comprising an AR-V7 specific junction(s). Primer pairs that can be used in accordance with the present invention preferably comprise at least one primer spanning one of the AR-V7 specific junctions or amplify a nucleotide fragment having an AR-V7 specific size due to the above described AR-V7 specific regions. Thus, primers that can be used in accordance with the present invention preferably only amplify AR-V7 coding cDNA (generated from mRNA coding for AR-V7) but not full-length AR or any other AR splice variant, such as e.g., AR-V1 to AR-V6. Preferred primer pairs of the present invention are disclosed in Table 1 herein and have a nucleotide sequences set forth in SEQ ID NOs: 1 and 2, SEQ ID NOs: 3 and 4, SEQ ID NOs: 1 and 4, SEQ ID NOs: 3 and 2, SEQ ID NOs: 1 and 5 and SEQ ID NOs: 3 and 5. Even more preferred primer pairs of the present invention have a nucleotide sequences set forth in SEQ ID NOs: 1 and 2, SEQ ID NOs: 3 and 2, SEQ ID NOs: 1 and 5 and SEQ ID NOs: 3 and 5.

In a further preferred embodiment, the result of the qualitative PCR is obtained by electrophoresis, preferably automated electrophoresis. A preferred automated electrophoresis system that can be used in accordance with the present invention is the Agilent - 2100 Bioanalyzer with DNA 1000 chip.

In a further preferred embodiment of the invention, the method further comprises verifying the AR-V7 status by sequencing the AR-V7 encoding nucleotide sequence. In general, any suitable sequencing technique known in the art can be used. Sequencing can be performed using either the cDNA obtained from the prostate cancer cell or the product obtained from the PCR using AR-V7 specific primers. In a preferred embodiment the product obtained from the PCR using AR-V7 specific primers is used for sequencing. Preferably, sequencing is performed with two different primers (e.g., primers having SEQ ID NO: 1 and SEQ ID NO: 4) in order to have at least two readings per nucleotide across two AR-V7 specific exon junctions, i.e., junction between AR-V7 exon 2 and exon 3 and junction between AR-V7 exon 3 and exon CE3. In a further preferred embodiment, sequencing is performed using at least one primer selected from the group consisting of SEQ ID NO: 1, SEQ ID NO: 2, SEQ ID NO: 3, SEQ ID NO: 4, and SEQ ID NO: 5.

The method of the invention allows stratifying a subject suffering from prostate cancer for the sensitivity and resistance to *abirateronelenzalutamide-* and taxane-based therapies. In a preferred embodiment of the invention, a subject positive for AR-V7 is stratified as being resistant to therapy with *abiraterone* and/or *enzalutamide.* Accordingly, a subject positive for AR-V7 is preferably treated with chemotherapy, more preferably with taxane-based chemotherapy. In a further preferred embodiment, a subject negative for AR-V7 is eligible to therapy with *abiraterone* and/or *enzalutamide.*

In a further preferred embodiment, the present invention provides a primer pair selected from the group consisting of primers having the nucleotide sequences set forth in SEQ ID NOs: 1 and 2, SEQ ID NOs: 3 and 4, SEQ ID NOs: 1 and 4, SEQ ID NOs: 3 and 2, SEQ ID NOs: 1 and 5 and SEQ ID NOs: 3 and 5. Even more preferred primer pairs of the present invention are selected from the group consisting of primers having a nucleotide sequences set forth in SEQ ID NOs: 1 and 2, SEQ ID NOs: 3 and 2, SEQ ID NOs: 1 and 5 and SEQ ID NOs: 3 and 5. The primers of the present invention have been designed to specifically amplify a fragment of a nucleotide sequence encoding AR-V7. Accordingly, primer pairs that can be used in accordance with the present invention preferably comprise at least one primer spanning one of the AR-V7 specific junctions. Thus, primers that can be used in accordance with the present invention provide a AR-V7 specific signal, i.e., the amplification product has a site that is characteristic for the AR-V7 specific regions described herein. Preferably the primers only amplify AR-V7 coding cDNA (generated from mRNA coding for AR-V7) but not full-length AR or any other AR splice variant, such as e.g., AR-V1 to AR-V6. However, such primers cannot be generated using a software without further ado but have to be designed based on the knowledge of AR-V7 specific regions.

The present invention further provides a kit comprising components that allow to put the present invention into practice. A kit according to the present invention comprises a primer pair selected from the group consisting of primers having the nucleotide sequences set forth in SEQ ID NOs: 1 and 2, SEQ ID NOs: 3 and 4, SEQ ID NOs: 1 and 4, SEQ ID NOs: 3 and 2, SEQ ID NOs: 1 and 5 and SEQ ID NOs: 3 and 5. In a preferred embodiment the kit of the invention comprises a primer pair selected from the group consisting of primers having a nucleotide sequences set forth in SEQ ID NOs: 1 and 2, SEQ ID NOs: 3 and 2, SEQ ID NOs: 1 and 5 and SEQ ID NOs: 3 and 5.

In a preferred embodiment, the kit further comprises a DNA polymerase. In a further preferred embodiment, the kit further comprises a buffer and/or reagents, such as e.g., dNTPs, for performing a PCR.

### V. Examples

The following Examples illustrate the invention, but are not to be construed as limiting the scope of the invention.

### Materials and Methods

Chemical Patients diagnosed with PC were recruited nationwide and they signed prior testing informed consent approved by the institutional Ethical Committee. Patients were examined by an urologist and their mCRPC status was confirmed biochemically and radiologically. Within several days of mCRPC confirmation and before initiation of consecutive therapies 6 ml of peripheral blood was collected into *Vacutainer®* tube (Becton, Dickinson and Company, Heidelberg, DE) containing K₂EDTA additive. These tubes were handled according to the manufacturer instructions and transferred to ice. 5 ml of whole blood was used no later than 4 hours after blood withdrawal for isolation of CTC using the AdnaTest® system consisting of the *ProstateCancerSelect* and the *ProstateCancerDetect* components. The *ProstateCancerSelect* component utilises magnetic beads coated with antibodies against surface antigens EpCAM and HER-2 to select prostate tissue-derived cells. Subsequently, from the CTC lysates polyA-rich RNAs were isolated using Oligo(dT)₂₅ included in the *ProstateCancerSelect* component, which were immediately converted to cDNA. cDNA libraries served for the detection of gene expression.

The gene expression panel included prostate-specific and tumour-associated genes such as *PSA, PSMA, EGFR* and AR alongside the actin gene, which served as an internal control. Generated RT-PCR products were analysed using the DNA 1000 chip on *Bioanalyser 2100* (Agilent/ Hermes, Bratislava, SK) according to the manufacturer instructions. Patients expressing at least one of the tested genes alongside the internal control were classified as CTC positive. Patients expressing actin but none of the tested genes were assigned a CTC negative status. AR-V7 expression was determined from cDNA library using sets of primers specified in the **Table 1.**

Primers set 1 and primer set 2 containing forward und reverse primers or their combinations consisting of one forward and one reverse primer (Generi Biotech, Hradec Kralove, CZ) were used to obtain the RT-PCR products. Similarly, one forward primer from primer set 1 or primer set 2 combined the singleton 3 primer (Generi Biotech, Hradec Kralove, CZ) were applied interchangeably to obtain the RT-PCR products. RT-PCR reaction mixture consisted of 1x concentrated PCR buffer [500 mM Tris/HCI (pH 8.3), 100 mM KCI, 50 mM (NH₄)₂SO₄], 1.5 mM MgCl₂, 0.2 mM dNTP and 1 U FastStart *Taq* DNA Polymerase (Roche, Bratislava, SK), and 0.8 µM of each primer. AdnaTest® system generated cDNA template of 3.2 ul was used in the final reaction volume of 20 ul. The cycling conditions included denaturation at 95°C for 4 min., 40 cycles of denaturation at 95°C for 30 sec., primer annealing at 60°C for 30 sec. and elongation at 72°C for 60 sec. and 1 cycle of elongation at 72°C for 7 min. and cooling down at 4 °C for infinite time. The presence or absence of AR-V7 expression was verified using the DNA 1000 chip on *Bioanalyser 2100* according to the manufacturer instructions.

The AR-V7 positive status of mCRPC patients was verified by Sanger sequencing using either of the aforementioned forward and reverse primers to obtain a minimum of 1 base coverages in one directions of the AR-V7 region spanning the junction between exon 2 and exon 3 and/or the junction between exon 3 and cryptic exon 3 (CE3). PCR and sequencing products were purified using standardised *NucleoSpin® Gel and PCR Clean-up* procedure (Macherey-Nagel/Biotech, Bratislava, SK) according to the manufacturer instructions. Cycle-sequencing using BigDye v3.1 (Applied Biosystems/Thermo Fischer Scientific, Bratislava, SK) and the PCR product-specific primer indicated in **Table 1** was performed according to the manufacturer instructions on MJ Mini Thermal Cycler (Applied Biosystems/Thermo Fischer Scientific, Bratislava, SK) and the sequencing products were resolved and analysed on the *Genetic Analyzer 3500* sequencer (Biorad, Bratislava, SK).

### Results

Using the AdnaTest® system 75 mCRPC patients were screened for the presence or absence of CTC (Figure 1). Both the mean age and the median age of mCRPC patients were 72 years with the two youngest patients having 55 year and the two oldest patients having 84 years. The median PSA level in these patients was 18.9 ng/ml, the Gleason score was ≥ 8 and the median time from diagnosis to development of CRPC was 28 months. mCRPC patients selected for this study were *abiraterone* and *enzalutamide* naïve with continuous ADT. Isolated CTC expressed on their surface EpCAM and/or HER-2 proteins and intracellularly tissue-specific genes such as *PSA, PSMA, EGFR* and/or AR. At least one surface protein and at least one intracellularly expressed gene were detected in 41 mCRPC patients (54.7 %), which is in line with the AdnaTest® system reported CTC distribution of between 33.3 % to 86.5 % with the 95 % confidence interval of 48 % to 75 % depending on the mCRPC patients' medical history (Roviello G et al., (2017). Onco Targets Ther., 10: 3811-3815; Capoun O et al., (2016). Anticancer Res., 36(4): 2019-26; Danila DC et al., (2016). Cancer J., 22(5): 315-320). Most frequently expressed genes in CTC positive patients were PSA in 39 patients (95.1 %) followed by expression of *AR, PSMA* and *EGFR* in 28 patients (68.3 %), 23 patients (56.1 %), and 7 patients (17.1 %), respectively (Table 2). These gene expression frequencies in CTC of our mCRPC patients are in agreement with those reported by Samoila *et al*. using the AdnaTest® system for *PSA, PSMA, EGFR* of 96.15 %, 53.85 % and 15.40 %, respectively (Samoila A *et al.,* A pilot study comparing sensitivity of detecting prostate cancer associated transcripts by AdnaTest Prostate Select/Detect assay to Veridex CellSearch in whole blood from patients with castration resistant prostate cancer (2012). Poster accessed at 14.07.2018 on http://www.adnagen.com). Since mCRPC is a clinically heterogeneous disease, it is argued that patients expressing *EGFR* have likely an endocrine component in their tumours and hence differ in the molecular subtype from those patients who are CTC positive and *EGFR* negative (Scher HI et al., (2017). Cancer Res., 77(20): 5687-5698). The subtle differences in the expression profiles of genes tested and untested in this study or their combination may influence the efficiency of therapies currently offered to mCRPC patients.

All patients, 41 CTC positive patients and 34 CTC negative patients, were also subjected to AR-V7 expression examination with CTC negative patients serving as and proving to be the biological negative control. From 41 CTC positive patients 12 patients (29.3 %) were found to be AR-V7 positive. AR-V7 positive status of mCRPC patients was confirmed by Sanger sequencing and found to be in 100 % agreement (Figure 2). It is also in line with the *AR-V7*/*AR-full length* qualitative expression ratio of 42.9 % calculated in this study and 38.0 % reported by other users of the AdnaTest® system that was coupled with ultrasensitive nanofluidic quantitative RT-PCR method for detection of *AR-V7* and *AR-full length* expression (Škereňová M et al., Gene Expression Analysis of Immunomagnetically Enriched Circulating Tumor Cell Fraction in Castration-Resistant Prostate Cancer (2018). Mol Diagn Ther., ahead of print). The *AR-V7* expression frequencies of 29.3 % in CTC positive mCRPC patients in our patients's group are in agreement with reported primary resistance of 20 to 40 % to *abiraterone* and *enzalutamide* in mCRPC patients (Antonarakis ES et al., (2014). N Engl J Med., 371(11): 1028-38; Scher HI et al., (2012). N Engl J Med., 367(13): 1187-97; de Bono JS et al., (2011). N Engl J Med., 364(21): 1995-2005). All AR-V7 positive patients also expressed AR (100 %) and *PSA* (100 %) followed by expression of *PSMA* and *EGFR* in 8 patients (66.7 %) and 3 patients (25.0 %), respectively (Table 2).

### Discussion

PC is a clinically heterogeneous disease affecting worldwide each year nearly one million men and alone in Europe it accounts yearly for nearly 420 000 diagnosed cases (Siegel RL et al., CA Cancer J Clin. (2018) 68(1): 7-30; SEER - Surveillance Epidemiology, and End Results Program. Prostate Cancer - Cancer Stat Facts (2018), from http://seer.cancer.gov/statfacts/html/prost.html, accessed on 19/07/2018). Usually, PC is an indolent disease of elderly men with progression time lasting more than a decade, hence significant number of patients die from other than malignant sequelae. In a proportion of patients, however, mCRPC develops which is often referred to as the lethal stage of the disease. Patients diagnosed with mCRPC require timely and efficient therapeutic interventions, since the disease can progress rapidly and hence reduce survival time significantly.

AR-V7 expression has been established as a biomarker stratifying mCRPC patients to *abirateronelenzalutamide* non-responders (Antonarakis ES et al., (2014). N Engl J Med., 371(11): 1028-38). These ARSi, in contrast to alternative chemotherapies, are formulated in capsules and hence easy and conveniently administered to those who qualify for this treatment. Molecular tests investigating the expression of AR-V7 in tumour cells have been established and require invasively accessed tissues, i.e., solid biopsies or more recently liquid biopsies such as CTC. CTC are convenient source of tumour cells since they can be obtained frequently at local practitioner at a fraction of time and costs of surgical procedures. More importantly, CTC are valuable source of heterogeneous malignant cells with distinct proliferating and disseminating properties. For these reasons CTC are becoming a resourceful clinical material that can be used for monitoring of mCRPC progression including AR-V7 typing. In the recent years, AdnaTest® system has been established as a reliable clinically applicable test to ascertain the presence or absence of CTC in mCRPC patients. Although the test has high specificity and sensitivity, its current formulation lacks the AR-V7 assay to evaluate ARSi responders among eligible mCRPC patients. Here, in this study, the present inventors provide such a test that is as sensitive and as reliable as the more sophisticated, laborious, time-consuming and expensive AR-V7 testing platforms (Zhang T, Armstrong AJ, (2016). Curr Oncol Rep., 18(3): 1-8; Lokhandwala PM et al., (2017). J Mol Diagn. 19(1): 115-125; Ma Y et al., (2016). Int J Mol Sci., 17(8): 1-11; Škereňová M et al., Gene Expression Analysis of Immunomagnetically Enriched Circulating Tumor Cell Fraction in Castration-Resistant Prostate Cancer (2018). Mol Diagn Ther., ahead of print; Robinson D et al., (2015). Cell, 161(5): 1215-1228; Welti J et al., (2016). Eur Urol., 70(4): 599-608; Qu Y et al., (2015). Sci Rep., 5 (7654): 1-6). It is based on a conventional PCR and similarly to the qualitative AdnaTest® system, i.e., with positive or negative outcome, and it ascertains the mCRPC patients the AR-V7 positive or the AR-V7 negative status. Coupled with the AdnaTest® system it would provide the clinician with CTC and AR-V7 status in a mCRPC patient within 2-3 days of blood withdrawal. Such testing algorithms would allow a quick decision making, in contrast to the tests described so far in the literature, regarding the most efficient therapy. Personnel trained in molecular biology is capable to operate the test and provide the clinicians with fast and reliable results regarding the CTC and AR-V7 status and hence speed up the therapy decision-making that is crucial for maintaining the quality of life and improving life expectancy in mCRPC patients.

The group of 75 mCRPC patients is among the largest being tested using the AdnaTest® system and reported in the literature (Antonarakis ES et al., (2014). N Engl J Med., 371(11): 1028-38; Škereňová M et al., Gene Expression Analysis of Immunomagnetically Enriched Circulating Tumor Cell Fraction in Castration-Resistant Prostate Cancer (2018). Mol Diagn Ther., ahead of print; Capoun O et al., (2016). Anticancer Res., 36(4): 2019-26). Since the AdnaTest® system users increased over the recent years, we anticipate that the developed AR-V7 test will be a valuable addition to the current testing algorithms. Additionally, it can be used also separately with other CTC testing platforms isolating living or fixated cells, in a form, that allows for prompt isolation of RNA. Also in these testing scenarios AR-V7 expression testing described in this study is easy and convenient to perform with the potential for clinical application.

### Tables

**Table 1: Primers used to detect expression of AR-V7.**

| Primer name | Sequence (5-3') | SEQ ID NOs |
|---|---|---|
| Set 1 | | |
| AR-V7.1 Fw | CACATGTGGAAGCTGCAAGGTC | SEQ ID NO: 1 |
| AR-V7.1 Rev | GCCAACCCGGAATTTTTCTCCC | SEQ ID NO: 2 |

| Set 2 | | |
|---|---|---|
| AR-V7.2 Fw | TGTGCGCCAGCAGAAATGATTG | SEQ ID NO: 3 |
| AR-V7.2 Rev | TTGAATGAGGCAAGTCAGCCTTTC | SEQ ID NO: 4 |

| Singleton 3 | | |
|---|---|---|
| AR-V7.3 Rev | CAGGCAAGTCAGGGAGAGTC | SEQ ID NO: 5 |

**Table 2. Summary on the CTC and AR-V7 statuses of tested mCRPC patients including tumour-associated gene expression profiles.**

| Patients n (%) | | CTC+ | PSA | PSMA | EGFR | AR | AR-V7 |
|---|---|---|---|---|---|---|---|
| All | 75 | 41 (54.7) | 39 (52.0) | 23 (30.7) | 7 (9.3) | 28 (37.3) | 12 (16.0) |
| CTC+ | 41 | 41 (100) | 39 (95.1) | 23 (56.1) | 7 (17.1) | 28 (68.3) | 12 (29.3) |
| AR-V7+ | 12 | 12 (100) | 12 (100) | 8 (66.7) | 3 (25.0) | 12 (100) | 12 (100) |

| | | | | | | | |
|---|---|---|---|---|---|---|---|
| CTC, circulating tumour cells +, positive | | | | | | | |

## Claims

1. A method for stratifying a subject suffering from prostate cancer, comprising:
(i) isolating a prostate cancer cell from a biopsy obtained from the subject;
(ii) obtaining mRNA from the prostate cancer cell;
(iii) preparing cDNA from the mRNA obtained in step (ii); and
(iv) performing a qualitative PCR to determine the AR-V7 status of the cell.

2. The method of claim 1, wherein the biopsy is a solid biopsy.

3. The method of claim 1, wherein the biopsy is a liquid biopsy, preferably the biopsy is peripheral blood.

4. The method of claim 1 or 3, wherein the prostate cancer cell is a circulating tumor cell,
wherein the circulating tumor cell is preferably isolated from the peripheral blood of the subject using magnetic beads coated with antibodies binding to EpCAM and HER-2; and/or
wherein the circulating tumor cell is identified by determining gene expression of PSA, PSMA, EGFR and/or AR, wherein a circulating tumor cell expresses at least one of said genes.

5. The method of any one of claims 1-4, wherein the prostate cancer is metastatic castration-resistant prostate cancer (mCRPC).

6. The method of any one of claims 1-5, wherein the qualitative PCR is not a quantitative PCR.

7. The method of any one of claims 1-6, wherein the qualitative PCR is performed using primers specifically amplifying AR-V7, wherein the primers preferably have a sequence set forth in SEQ ID NOs: 1 and 2, SEQ ID NOs: 3 and 4 or combinations thereof or a combination of SEQ ID NO: 1 or 3 with SEQ ID NO: 5.

8. The method of any one of claims 1-7, wherein the result of the qualitative PCR is obtained by electrophoresis, preferably automated electrophoresis.

9. The method of claims 1-8, wherein the method further comprises step
(v) verifying the AR-V7 status by sequencing,
wherein sequencing is preferably performed using the primers defined in claim 7.

10. The method of any one of claims 1-9, wherein a subject positive for AR-V7 is stratified as being resistant to therapy with *abiraterone* and/or *enzalutamide .*

11. The method of claim 13, wherein the subject is treated with chemotherapy, preferably taxane-based chemotherapy.

12. The method of any one of claims 1-11, wherein a subject negative for AR-V7 is eligible to therapy with *abiraterone* and/or *enzalutamide.*

13. A primer pair selected from the group consisting of primers having the sequences set forth in SEQ ID NOs: 1 and 2, SEQ ID NOs: 3 and 4, SEQ ID NOs: 1 and 4, SEQ ID NOs: 3 and 2, SEQ ID NOs: 1 and 5 and SEQ ID NOs: 3 and 5.

14. A kit comprising at least one primer pair as defined in claim 13.

15. The kit of claim 14 further comprising a DNA polymerase and optionally buffers and reagents for performing a PCR.


SEQUENCE LISTING

<110>  Jessenius Faculty of Medicine of Comenius University in Martin

<120>  Means and methods for stratifying prostate cancer patients

<130>  CUB16462EP

<160>  6

<170>  PatentIn version 3.5

<210>  1
<211>  22
<212>  DNA
<213>  Artificial Sequence

<220>
<223>  primer sequence

<400>  1
cacatgtgga agctgcaagg tc                                                22

<210>  2
<211>  22
<212>  DNA
<213>  Artificial Sequence

<220>
<223>  primer sequence

<400>  2
gccaacccgg aatttttctc cc                                                22

<210>  3
<211>  22
<212>  DNA
<213>  Artificial Sequence

<220>
<223>  primer sequence

<400>  3
tgtgcgccag cagaaatgat tg                                                22

<210>  4
<211>  24
<212>  DNA
<213>  Artificial Sequence

<220>
<223>  primer sequence

<400>  4
ttgaatgagg caagtcagcc tttc                                              24

<210>  5
<211>  20
<212>  DNA

<213>  Artificial Sequence

<220>
<223>  primer sequence

<400>  5
caggcaagtc agggagagtc                                                   20


<210>  6
<211>  3641
<212>  DNA
<213>  Homo sapiens

<400>  6
gacactgaat ttggaaggtg gaggattttg tttttttctt ttaagatctg ggcatctttt      60

gaatctaccc ttcaagtatt aagagacaga ctgtgagcct agcagggcag atcttgtcca     120

ccgtgtgtct tcttctgcac gagactttga ggctgtcaga gcgctttttg cgtggttgct     180

cccgcaagtt tccttctctg gagcttcccg caggtgggca gctagctgca gcgactaccg     240

catcatcaca gcctgttgaa ctcttctgag caagagaagg ggaggcgggg taagggaagt     300

aggtggaaga ttcagccaag ctcaaggatg gaagtgcagt tagggctggg aagggtctac     360

cctcggccgc cgtccaagac ctaccgagga gctttccaga atctgttcca gagcgtgcgc     420

gaagtgatcc agaacccggg ccccaggcac ccagaggccg cgagcgcagc acctcccggc     480

gccagtttgc tgctgcagca gcagcagcag cagcagcagc agcagcagca gcagcagcag     540

cagcagcagc agcagcagca gcagcagcag cagcagcagc aagagactag ccccaggcag     600

cagcagcagc agcagggtga ggatggttct ccccaagccc atcgtagagg ccccacaggc     660

tacctggtcc tggatgagga acagcaacct tcacagccgc agtcggccct ggagtgccac     720

cccgagagag gttgcgtccc agagcctgga gccgccgtgg ccgccagcaa ggggctgccg     780

cagcagctgc cagcacctcc ggacgaggat gactcagctg ccccatccac gttgtccctg     840

ctgggcccca ctttccccgg cttaagcagc tgctccgctg accttaaaga catcctgagc     900

gaggccagca ccatgcaact ccttcagcaa cagcagcagg aagcagtatc cgaaggcagc     960

agcagcggga gagcgaggga ggcctcgggg gctcccactt cctccaagga caattactta    1020

gggggcactt cgaccatttc tgacaacgcc aaggagttgt gtaaggcagt gtcggtgtcc    1080

atgggcctgg gtgtggaggc gttggagcat ctgagtccag gggaacagct tcggggggat    1140

tgcatgtacg ccccactttt gggagttcca cccgctgtgc gtcccactcc ttgtgcccca    1200

ttggccgaat gcaaaggttc tctgctagac gacagcgcag gcaagagcac tgaagatact    1260

gctgagtatt cccctttcaa gggaggttac accaaagggc tagaaggcga gagcctaggc    1320

tgctctggca gcgctgcagc agggagctcc gggacacttg aactgccgtc taccctgtct    1380

ctctacaagt ccggagcact ggacgaggca gctgcgtacc agagtcgcga ctactacaac    1440

tttccactgg ctctggccgg accgccgccc cctccgccgc ctccccatcc ccacgctcgc 1500

atcaagctgg agaacccgct ggactacggc agcgcctggg cggctgcggc ggcgcagtgc 1560

cgctatgggg acctggcgag cctgcatggc gcgggtgcag cgggacccgg ttctgggtca 1620

ccctcagccg ccgcttcctc atcctggcac actctcttca cagccgaaga aggccagttg 1680

tatggaccgt gtggtggtgg tgggggtggt ggcggcggcg gcggcggcgg cggcggcggc 1740

ggcggcggcg aggcgggagc tgtagccccc tacggctaca ctcggccccc tcaggggctg 1800

gcgggccagg aaagcgactt caccgcacct gatgtgtggt accctggcgg catggtgagc 1860

agagtgccct atcccagtcc cacttgtgtc aaaagcgaaa tgggcccctg gatggatagc 1920

tactccggac cttacgggga catgcgtttg gagactgcca gggaccatgt tttgcccatt 1980

gactattact ttccacccca gaagacctgc ctgatctgtg gagatgaagc ttctgggtgt 2040

cactatggag ctctcacatg tggaagctgc aaggtcttct tcaaaagagc cgctgaaggg 2100

aaacagaagt acctgtgcgc cagcagaaat gattgcacta ttgataaatt ccgaaggaaa 2160

aattgtccat cttgtcgtct tcggaaatgt tatgaagcag ggatgactct gggagaaaaa 2220

ttccgggttg gcaattgcaa gcatctcaaa atgaccagac cctgaagaaa ggctgacttg 2280

cctcattcaa aatgagggct ctagagggct ctagtggata gtctggagaa acctggcgtc 2340

tgaggcttag gagcttaggt ttttgctcct caacacagac tttgacgttg gggttggggg 2400

ctactctctt gattgctgac tccctccagc gggaccaata gtgttttcct acctcacagg 2460

gatgttgtga ggacgggctg tagaagtaat agtggttacc actcatgtag ttgtgagtat 2520

catgattatt gtttcctgta atgtggcttg gcattggcaa agtgcttttt gattgttctt 2580

gatcacatat gatgggggcc aggcactgac tcaggcggat gcagtgaagc tctggctcag 2640

tcgcttgctt ttcgtggtgt gctgccagga agaaactttg ctgatgggac tcaaggtgtc 2700

accttggaca agaagcaact gtgtctgtct gaggttcctg tggccatctt tatttgtgta 2760

ttaggcaatt cgtatttccc ccttaggttc tagccttctg gatcccagcc agtgacctag 2820

atcttagcct caggccctgt cactgagctg aaggtagtag ctgatccaca gaagttcagt 2880

aaacaaggac cagatttctg cttctccagg agaagaagcc agccaacccc tctcttcaaa 2940

cacactgaga gactacagtc cgactttccc tcttacatct agccttactg tagccacact 3000

ccttgattgc tctctcacat cacatgcttc tcttcatcag ttgtaagcct ctcattcttc 3060

tcccaagcca gactcaaata ttgtattgat gtcaaagaag aatcacttag agtttggaat 3120

atcttgttct ctctctgctc catagcttcc atattgacac cagtttcttt ctagtggaga 3180

agtggagtct gtgaagccag ggaaacacac atgtgagagt cagaaggact ctccctgact 3240

tgcctggggc ctgtctttcc caccttctcc agtctgtcta aacacacaca cacacacaca 3300

cacacacaca cacacacaca cacacgctct ctctctctct ccccccccaa cacacacaca 3360

ctctctctct cacacacaca cacatacaca cacacttctt tctctttccc ctgactcagc 3420

aacattctgg agaaaagcca aggaaggact tcaggagggg agtttccccc ttctcagggc 3480

agaattttaa tctccagacc aacaagaagt tccctaatgt ggattgaaag gctaatgagg 3540

tttattttta actactttct atttgtttga atgttgcata tttctactag tgaaattttc 3600

ccttaataaa gccattaata cacccaaaaa aaaaaaaaaa a 3641